# FASCICULE DE BREVET EUROPEEN

(11) **EP 0 998 915 B1**
(45) Date de publication et mention de la délivrance du brevet: **09.06.2004**
(21) Numéro de dépôt: 99401955.2
(22) Date de dépôt: 30.07.1999
(51) Int. Cl.: A61K 7/48

(54) **Utilisation d'une gomme de silicone pour stabiliser l'acide ascorbique, et nouvelles compositions contenant ces composants**
Gebrauch von Silicongummi zur Stabilisierung von Ascorbinsaüre und Vorbereitungen die diese Zusammensetzungen enthalten
Use of silicon gum to stabilize ascorbic acid, and new compositions containing these compounds

(30) Priorité: 29.09.1998 FR 9812156
(43) Date de publication de la demande: 10.05.2000
(73) Titulaire: L'OREAL, 75008 Paris (FR)
(72) Inventeur: Afriat, Isabelle, 75003 Paris (FR)
(74) Mandataire: Rasson, Catherine

(56) Documents cités:
- EP-A- 0 670 157
- EP-A- 0 672 410
- EP-A- 0 755 674
- WO-A-94/09756
- WO-A-96/24325
- WO-A-98/00102
- WO-A-98/34591
- US-A- 5 391 373
- US-A- 5 891 470
- "Forever young firming eye gel" PRODUCT INFORMATION SHEET N. 8739,1999, pages 1-4, XP002105965 www.cybervitamins.com/eyegel.htm

## Description

L'invention se rapporte à l'utilisation d'une gomme de silicone pour stabiliser l'acide ascorbique et à de nouvelles compositions contenant ces composants, utilisables en particulier dans les domaines cosmétique, et dermatologique.

L'invention se rapporte aussi à une utilisation de ces compositions pour le traitement cosmétique de la peau ainsi que pour la préparation d'une crème ou pommade destinée au traitement dermatologique de la peau.

L'invention se rapporte encore à un procédé de traitement cosmétique qui consiste à appliquer sur la peau lesdites compositions.

Les compositions de l'invention peuvent être appliquées, par voie topique, sur le visage, y compris autour des yeux, sur le corps ainsi que sur le cuir chevelu des êtres humains.

On cherche depuis longtemps à stabiliser l'acide ascorbique ou vitamine C, dans des présentations galéniques appropriées du fait de ses propriétés bénéfiques. En effet, l'acide ascorbique possède de nombreuses fonctions biologiques comme la stimulation de la synthèse du collagène, le renfort des tissus cutanés contre les agressions extérieures (rayonnements UV, pollution), la dépigmentation, l'activité anti-radicaux libres, la compensation de la déficience en vitamine E. Certaines de ces propriétés bénéfiques ont été rapportées notamment par ENGLAND ET SEIFTER dans l'article "The biochemical functions of ascorbic acid" paru dans Ann. Rev. Nutri., 1986 : 6, pp 365-406.

Cependant, en raison de sa structure chimique (d'alpha-cétolactone), l'acide ascorbique est très sensible à l'influence des paramètres de l'environnement comme la lumière, l'oxygène, l'eau (par son pH et par la présence de trace de métaux). Il s'ensuit une dégradation inéluctable au cours du temps de l'acide ascorbique en solution. En outre, les émulsions contenant de l'acide ascorbique ont tendance à être instables, c'est à dire à se séparer en deux phases au cours du temps.

Dans l'état de la technique ce problème a été diversement traité. Pour diminuer ou retarder la dégradation de l'acide ascorbique en solution, les auteurs du document US-A-5140043 ont préconisé de le stabiliser en l'introduisant dans des solutions hydroalcooliques, formées d'au moins 80% d'eau et ayant un pH inférieur à 3,5. En raison de la forte acidité de ces solutions, leur utilisation dans le domaine cosmétique et/ou pharmaceutique est difficilement envisageable. En effet, une application répétée de ces solutions peut perturber l'équilibre de la peau et en particulier irriter, voir brûler la peau.

On connaît, par ailleurs, l'article de B.R. Hajratwala intitulé "Stability of ascorbic acid", paru dans la Revue Sciences Pharmaceutiques, le 15 mars 1985. Dans cet article, il est enseigné notamment de stabiliser l'acide ascorbique en solution aqueuse acide par ajout d'un agent tensioactif qui est un ester de sorbitane oxyéthyléné. En particulier, l'auteur y a rapporté qu'à pH 3,4 et à 25°C, l'ajout de cet agent diminuait la vitesse d'oxydation, donc de dégradation de l'acide ascorbique en solution. En outre, ce document enseigne l'emploi d'agent chélatant tel que l'éthylène diamine tétraacétique (EDTA) et le conditionnement sous azote, en l'absence de lumière, pour améliorer la stabilité de l'acide ascorbique en solution aqueuse. Une telle solution aqueuse acide, appliquée sur la peau, présente les mêmes inconvénients que ceux décrits ci-dessus pour des solutions hydroalcooliques acides. De plus, la stabilisation obtenue est encore insuffisante.

D'autres modes de stabilisation de l'acide ascorbique ont été envisagés notamment par enrobage (technique décrite dans le document FR-A-1600826) ou par granulation de l'acide ascorbique (technique illustrée dans le document JP-A-53-127819, pour l'agro-alimentaire). Mais ces techniques sont d'une part coûteuses et peuvent d'autre part altérer l'acide ascorbique, par exemple lors d'un chauffage, et/ou conduire à des compositions peu cosmétiques, comme c'est le cas des granulés.

On connaît, par ailleurs, du document FR-A-1489249 l'emploi de sels métalliques d'acide ascorbique phosphorylé, notamment l'ascorbylphosphate de magnésium, dans des compositions cosmétiques. Ce dernier composé a une activité proche de celle de l'acide ascorbique dont il est issu mais il présente certains inconvénients qui rendent son utilisation sur la peau peu probable. En particulier, l'ascorbylphosphate de magnésium n'étant stable qu'en pH basique (pH 8 à pH 9), il doit être incorporé dans une composition basique qui peut être irritante pour la peau (dont le pH a une valeur d'environ 5,5).

Le document EP-A--0 670 157 décrit une émulsion comprenant une phase aqueuse contenant de l'acide ascorbique et présentant un pH acide, d'une valeur au plus égale à 3,5.

Des compositions à base de polyols d'acide carboxylique combinés à des émollients spécifiques et à de l'acide ascorbique sont décrites dans le document WO 98/22075. Le pH préconisé de ces compositions est compris entre 7 et 8. De tels pH acide ou basique peuvent présenter des inconvénients.

En conséquence, l'ensemble des propositions qui ont été faites jusqu'ici n'a pas permis de résoudre les problèmes techniques liés à l'instabilité de l'acide ascorbique en solution, dans une forme galénique appropriée alliant efficacité et confort pour les domaines cosmétiques et/ou dermatologiques et à un coût compatible avec les exigences industrielles.

Il subsiste donc le besoin d'une composition, utilisable dans les domaines cosmétique, dermatologique contenant de l'acide ascorbique stabilisé, à l'état libre, c'est à dire sans groupement additionnel notamment stabilisant, et qui ne provoque aucune irritation de la peau, après application.

Le document FR-1 600 826 décrit la stabilisation de l'acide ascorbique sous forme solide par enrobage à l'aide de polymère siloxane, lors de la fabrication de comprimés.

La demande de brevet WO-98/00102 divulgue des compositions dans lesquelles l'acide ascorbique est stabilisé par le diméthyl isosorbide. Une huile siliconée non réticulée peut être ajoutée à la composition pour améliorer son aspect et renforcer sa stabilité.

Le document EP 0 704 205 décrit des compositions anhydres de maquillage contenant une gomme de silicone. Ces compositions sont résistantes à l'eau et présentent une bonne homogénéité.

La demanderesse a maintenant découvert, de façon surprenante, que l'utilisation d'une gomme de silicone permettait de stabiliser l'acide ascorbique. L'intérêt d'utiliser une gomme de silicone réside notamment dans le fait de pouvoir utiliser l'acide ascorbique, dans une composition ne provoquant pas d'irritation ni de brûlure de la peau. La composition de l'invention est par conséquent bien supportée par les utilisateurs. En outre, la composition de l'invention présente l'avantage d'être confortable sans être grasse.

De plus, le fait que la composition de l'invention puisse incorporer de l'acide ascorbique à l'état libre, protoné, procure des traitements plus efficaces par rapport aux préparations de l'art antérieur qui comportent des "dérivés" d'acide ascorbique hydrolysables au contact de la peau.

Les compositions de l'invention peuvent se présenter notamment sous forme de lait ou de crèmes utilisables en particulier dans les domaines cosmétique, dermatologique. Elles possèdent une texture légère et s'étalent bien. Elles donnent de plus, à l'application, une sensation de fraîcheur et procurent un éclat du teint instantané. Elles permettent, en particulier, un lissage des traits et des imperfections de la peau.

D'autre part, les compositions conformes à l'invention présentent l'avantage de pouvoir inclure tout type d'actif hydrophile ou lipophile, le pH des compositions étant neutre ou faiblement acide.

Par ailleurs, les compositions de l'invention présentent de bonnes propriétés de stabilité, notamment à température ambiante (20° C).

La présente invention a donc pour objet essentiel l'utilisation d'une gomme de silicone pour stabiliser l'acide ascorbique ou l'un de sels dans une composition topique.

Dans le cadre de la présente invention, on entend par "acide ascorbique" ou vitamine C, l'acide ascorbique à l'état libre, protoné ou l'un de ses sels.

Dans le cadre de la présente invention, on désigne sous le terme "gomme de silicone" des polydiméthyl siloxanes linéaires non réticulés qui peuvent être hydroxylés ou phénylés et qui ont la consistance d'une huile épaisse ou d'un solide transparent par opposition avec les alkyl, alkoxy diméthicones qui, lorsqu'ils sont solides, présentent un aspect opaque cireux, mais peuvent également avoir l'aspect d'une huile limpide lorsque leur point de fusion est inférieur à la température ambiante.

La gomme de silicone peut être choisie parmi des polydiorganosiloxanes de masse moléculaire comprise entre 100.000 à 2.000.000, et préférentiellement entre 100.000 et 1.500.000.

Ces gommes de silicone présentent préférentiellement une viscosité égale ou supérieure à 200.000 cSt (0,2 m2/s) et préférentiellement supérieure à 300.000 cSt (0,3 m2/s), viscosité mesurée au viscosimètre BROOKFIELD à 25°C.

On utilise plus particulièrement la gomme de silicone choisie parmi les gommes de silicone de formule (I) suivante: 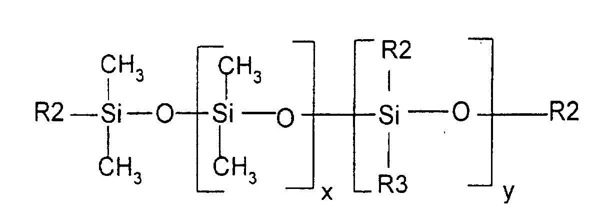 dans laquelle :
R2 représente -CH₃, -OH ou -C₆H₅,
R3 représente -CH₃, -OH, -C₆H₅ ou -OSi(CH₃)₃,
x = 0 ou un nombre entier et
y est un nombre entier sous réserve que y ou x et y soient des nombres entiers tels que la masse moléculaire moyenne en poids soit supérieure à 100.000, et de préférence comprise entre 100.000 et 1.500.000.
Les gommes de silicone préférées de l'invention sont choisies parmi les diméthicones (polydiméthylsiloxanes) et les diméthiconols (polydiméthylsiloxanes à terminaison hydroxyle).

La gomme de silicone peut être utilisée seule ou en mélange, notamment avec un solvant choisi parmi les silicones volatiles, les huiles polydiméthylsiloxanes, les huiles polyphénylméthylsiloxanes, les isoparaffines, le chlorure de méthylène, le pentane, le dodécane, le tridécane, le tétradécane ou leurs mélanges.

Les gommes de silicone sont généralement commercialisées par les fournisseurs sous forme présolubilisée ou non dans une proportion de 5 à 20 %, et de préférence de 10 à 15 % en poids, dans un polydiméthylsiloxane, linéaire ou cyclique, de faible poids moléculaire, volatile ou non volatile.

On peut citer à titre d'exemple en tant que gommes de silicone, la diméthicone à 96% dans du cyclométhicone vendue par la Société RHONE POULENC sous la dénomination de "Mirasil DM-500000®", et la diméthicone vendue par la société WACKER sous la dénomination "AK 300000®", et les diméthiconols vendus sous les dénominations de "Q2-1403®", et "Q2-1401®" et "Q2-1503®" par la Société DOW CORNING.

Un autre objet de l'invention est une composition topique sous forme d'émulsion comprenant de l'acide ascorbique ou l'un de ses sels, une gomme de silicone et un émulsionnant siliconé et présentant un pH compris entre 4 et 7, et de préférence entre 5,5 et 6,5.

Les compositions selon l'invention peuvent notamment consister en une émulsion eau-dans-huile ou huile-dans-eau.

Les gommes de silicone sont celles décrites ci-dessus. De même, la gomme de silicone peut être utilisée seule ou en mélange avec un solvant choisi parmi les silicones volatiles, les huiles polydiméthylsiloxanes, les huiles polyphénylméthylsiloxanes ou leurs mélanges.

L'acide ascorbique est présent dans les compositions de l'invention dans une concentration allant de 0,1 à 20 % et de préférence de 2 à 10 % en poids par rapport au poids total de la composition.

La gomme de silicone est présente dans les compositions de l'invention dans une concentration en matière active allant de 1 à 20 % et de préférence de 2 à 15 % en poids par rapport au poids total de la composition.

Dans une forme de réalisation particulièrement préférée de l'invention, la composition contient en outre au moins un polyol. Le polyol peut être choisi parmi la glycérine, le sorbitol ou les glycols, en particulier le propylène glycol, le butylène glycol et les polyéthylène glycols.

Selon un mode de réalisation particulier de l'invention, le ou les polyols se trouvent totalement ou partiellement sous forme complexée avec un polymère acrylique ou méthacrylique. Le polymère peut également comprendre de l'eau liée, c'est-à-dire être complexé avec un mélange d'eau et de polyol(s). On entend par polymère acrylique ou méthacrylique, un homopolymère ou un copolymère d'acide acrylique ou méthacrylique ou un homopolymère ou un copolymère d'un dérivé d'acide acrylique ou méthacrylique.

On peut citer comme homopolymère complexant l'eau et les polyols, ceux vendus sous les dénominations de Norgel et de Lubrajel CG par la société Guardian. Ces polymères sont des polyacrylates de glycéryle complexés avec plus de 65 % en poids de glycérine et/ou de propylène glycol et moins de 35 % en poids d'eau liée. Ces polymères apportent le polyol et l'eau complexés, et éventuellement jouent en outre le rôle de gélifiant de la composition.

Le polyol peut être présent dans une concentration allant de 5 à 40 % et de préférence de 15 à 30 % en poids par rapport au poids total de la composition.

Les compositions selon l'invention peuvent contenir en outre au moins une huile siliconée. Ces dernières peuvent notamment être choisies dans le groupe comprenant les silicones volatiles telles que le cyclopentadiméthylsiloxane et le cyclotétradiméthylsiloxane, les polydiméthylsiloxanes, les polyphényltriméthylsiloxanes et les silicones fluorées.

L'huile siliconée peut être présente dans une concentration allant de 2 à 40 % et de préférence de 10 à 35 % en poids par rapport au poids total de la composition.

Les compositions selon l'invention contiennent aussi en outre au moins un émulsionnant siliconé.

Les émulsionnants siliconés peuvent être choisis parmi les diméthicones copolyols et les alkyldiméthicone copolyols. On peut citer comme émulsionnant utilisable dans les compositions de l'invention le mélange de polyglycérol-4 isostéarate/cetyl diméthicone copolyol/hexyl laurate vendu sous la dénomination "Abil WE 09®" par la Société Goldschmidt, le cétyl diméthicone copolyol vendu sous la dénomination "Abil EM 90®" par la Société Goldschmidt et le mélange de cyclométhicone/diméthicone copolyol vendu sous la dénomination "Q2-3225C" ou "Q2-5225C"par la Société Dow Corning.

Selon un mode préféré de réalisation de l'invention, on utilise un alkyldiméthicone copolyol et notamment le cétyldiméthicone copolyol. L'émulsionnant est présent en une quantité allant de préférence de 0,1 à 10 % et mieux de 0,5 à 5 % du poids total de la composition.

Les compositions selon l'invention peuvent contenir en outre un ou plusieurs corps gras choisis parmi les cires ou résines de silicone, les huiles fluorées, les huiles d'origine animale, les huiles d'origine végétale, les huiles minérales ou les huiles synthétiques. Comme cires, on peut utiliser notamment les cires de silicone telles que les alcoxydiméthylsiloxanes, et plus particulièrement les stéaroxypolydiméthyl-siloxanes, les alkylpolysiloxanes et les polydiméthylsiloxanes à fonction mercapto. Comme résines, on peut utiliser notamment les résines de silicone telles que les triméthylsiloxysilicates.

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles en plus de l'acide ascorbique, les conservateurs, les antioxydants, les solvants, les parfums, les charges, les filtres, les absorbeurs d'odeur, les matières colorantes et les vésicules lipidiques. Les quantités de ces différents adjuvants sont celles classiquement utilisées dans les domaines considérés, et par exemple de 0,01% à 15% du poids total de la composition.

Comme actifs hydrophiles, on peut utiliser par exemple les protéines ou les hydrolysats de protéine, les acides aminés, l'allantoïne, les sucres et les dérivés de sucre, l'amidon, l'acide hyaluronique ou des extraits végétaux tels que des extraits de Ginkgo biloba ou des extraits de Ginseng.

Comme actifs lipophiles, on peut utiliser par exemple le tocophérol (vitamine E) et ses dérivés, les acides gras essentiels, les céramides, les huiles essentielles.

Comme charges, on peut citer la poudre de nylon et les microsphères (Acrylates copolymer).

Les compositions selon l'invention peuvent ainsi constituer une composition cosmétique et/ou dermatologique.

L'invention a encore pour objet une utilisation de la composition ci-dessus pour un traitement cosmétique de la peau et en particulier en vue de la tonifier, la régénérer, de lisser les rides et/ou les ridules de la peau, d'éclaircir le teint, d'éliminer les taches pigmentaires de la peau, et/ou pour lutter contre les méfaits des rayonnements UV, et/ou pour renforcer les tissus cutanés contre les agressions de l'environnement (pollution).

L'invention a encore pour objet l'utilisation d'une composition ci-dessus pour la fabrication d'une crème destinée à un traitement dermatologique.

L'invention a enfin pour objet un procédé de traitement cosmétique, consistant à appliquer sur la peau, y compris autour des yeux, une composition conforme à l'invention.

D'autres avantages et caractéristiques de l'invention apparaîtront mieux à la lecture des exemples qui suivent donnés à titre illustratif.

### Exemple 1 : Emulsion Eau dans huile

### Phase A

- Eau déminéralisée 25,05 %
- Glycérine 24 %
- Propylène glycol 7 %
- Conservateur 0,2 %
- Vitamine C 5 %
- Sulfate de magnésium 2 %
- Acrylates copolymer (Microsphères) 0,75 %

### Phase B :

- DC2 5225C (émulsionnant siliconé dans huile de silicone) 8 %
- Huile de silicone 20 cSt 23 %
- Silicone AK 300000 (gomme de silicone) 5 %

Le mode opératoire pour préparer cette composition est le suivant: :
On prépare chaque phase séparément et on introduit la phase A dans la phase B sous agitation.

On obtient une crème blanche, brillante et stable. Cette composition est apte à lisser les traits de la peau du visage et à éclaircir le teint.

### Exemple 2 : Emulsion eau dans huile

### Phase A :

- Eau déminéralisée 25,05 %
- Glycérine 24 %
- Propylène glycol 7 %
- Conservateur 0,2 %
- Vitamine C 5 %
- Sulfate de magnésium 2 %
- Acrylates copolymer 0,75 %

### Phase B

- Cétyldiméthicone copolyol (Abil EM90) 0,8 %
- Huile de silicone 20 cSt 23 %
- Cyclométhicone 7,2 %
- AK 300000 (gomme de silicone) 5 %

Le mode opératoire est le même que dans l'exemple 1.

On obtient une crème blanche, brillante et stable. Cette composition est apte à lisser les traits de la peau et à éclaircir le teint.

### Exemple 2 comparatif: Emulsion eau dans huile

### Phase A :

- Eau déminéralisée 25,05 %
- Glycérine 24 %
- Propylène glycol 7 %
- Conservateur 0,2 %
- Vitamine C 5 %
- Sulfate de magnésium 2 %
- Acrylates copolymer 0,75 %

### Phase B :

- Cétyldiméthicone copolyol (Abil EM90) 0,8 %
- Huile de silicone 20 cSt 28 %
- Cyclométhicone 7,2 %

Le mode opératoire est le même que dans l'exemple 1. On obtient une crème épaisse, qui est instable et se sépare rapidement en deux phases.

### Exemple 3 : Emulsion Eau dans huile

### Phase A :

- Eau déminéralisée 20 %
- Glycérine 24 %
- Propylène glycol 13 %
- Vitamine C 5 %
- Sulfate de magnésium 2 %

### Phase B :

- Cétyldiméthicone copolyol (Abil EM90) 0,8 %
- Huile de silicone 30,2 %
- Mirasol DM-500000 (gomme de silicone) 5 %

Le mode opératoire est identique à celui de l'exemple 1.

On obtient une crème blanche, stable, apte à lisser les traits de la peau et à éclaircir le teint.

## Revendications

1. Utilisation d'une gomme de silicone choisie parmi les polydiméthylsiloxanes linéaires non réticulés éventuellement hydroxylés ou phénylés pour stabiliser l'acide ascorbique ou l'un de ses sels dans une composition topique consistant en une émulsion eau-dans-huile ou huile-dans-eau.

2. Utilisation selon la revendication 1, **caractérisée en ce que** la gomme de silicone est choisie parmi des polydiorganosiloxanes de masse moléculaire comprise entre 100.000 à 2.000.000, et préférentiellement entre 100.000 et 1.500.000.

3. Utilisation selon la revendication 1 ou 2, **caractérisée en ce que** la gomme de silicone présente une viscosité égale ou supérieure à 0,2 m²/s et préférentiellement égale ou supérieure à 0,3 m²/s.

4. Utilisation selon l'une quelconque des revendications 1 à 3, **caractérisée en ce que** la gomme de silicone est choisie parmi les gommes de silicone de formule (I) suivante: 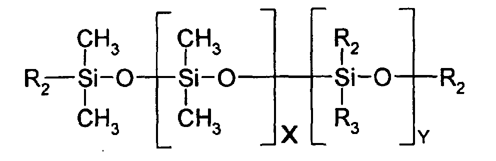 dans laquelle:
R₂ représente -CH₃, -OH ou -C₆H₅,
R₃ représente -CH₃, -OH, -C₆H₅ ou -OSi(CH₃)₃,
x = 0 ou un nombre entier et
y est un nombre entier sous réserve que y ou x et y soient des nombres entiers tels que la masse moléculaire moyenne en poids soit supérieure à 100.000, et de préférence comprise entre 100.000 et 1.500.000.

5. Utilisation selon l'une quelconque des revendications 1 à 4, **caractérisée en ce que** la gomme de silicone est choisie parmi les diméthicones et les diméthiconols.

6. Utilisation selon l'une quelconque des revendications 1 à 5, **caractérisée en ce que** la gomme de silicone est utilisée seule ou en mélange avec un solvant choisi parmi les silicones volatiles, les huiles polydiméthylsiloxanes, les huiles polyphénylméthylsiloxanes ou leurs mélanges.

7. Composition topique sous forme d'une émulsion eau-dans-huile ou huile-dans-eau, **caractérisée en ce qu'**elle comprend de l'acide ascorbique ou l'un de ses sels, une gomme de silicone choisie parmi les polydiméthylsiloxanes linéaires non réticulés éventuellement hydroxylés ou phénylés, un émulsionnant siliconé et présente un pH compris entre 4 et 7.

8. Composition selon la revendication 7, **caractérisée en ce que** la gomme de silicone est choisie parmi des polydiorganosiloxanes de masse moléculaire comprise entre 100.000 à 2.000.000, et préférentiellement entre 100.000 et 1.500.000.

9. Composition selon la revendication 7 ou 8, **caractérisée en ce que** la gomme de silicone présente une viscosité égale ou supérieure à 0,2 m²/s et préférentiellement égale ou supérieure à 0,3 m²/s.

10. Composition selon l'une quelconque des revendications 7 à 9, **caractérisée en ce que** la gomme de silicone est choisie parmi les gommes de silicone de formule (I) suivante: 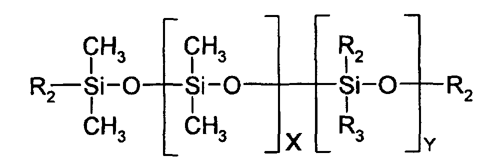 dans laquelle:
R₂ représente -CH₃, -OH ou -C₆H₅,
R₃ représente -CH₃, -OH, -C₆H₅ ou -OSi(CH₃)₃,
x = 0 ou un nombre entier et
y est un nombre entier sous réserve que y ou x et y soient des nombres entiers tels que la masse moléculaire moyenne en poids soit supérieure à 100.000, et de préférence comprise entre 100.000 et 1.500.000.

11. Composition selon l'une quelconque des revendications 7 à 10, **caractérisée en ce que** la gomme de silicone est choisie parmi les diméthicones et les diméthiconols.

12. Composition selon l'une quelconque des revendications 7 à 11, **caractérisée en ce que** la gomme de silicone est utilisée seule ou en mélange avec un solvant choisi parmi les silicones volatiles, les huiles polydiméthylsiloxanes, les huiles polyphénylméthylsiloxanes ou leurs mélanges.

13. Composition selon l'une quelconque des revendications 7 à 12, **caractérisée en ce que** l'acide ascorbique ou l'un de ses sels est présent dans une concentration allant de 0,1 à 20% et de préférence de 2 à 10% en poids par rapport au poids total de la composition.

14. Composition selon l'une quelconque des revendications 7 à 13, **caractérisée en ce que** la gomme de silicone est présente dans une concentration en matière active allant de 1 à 20% et de préférence de 2 à 15% en poids par rapport au poids total de la composition.

15. Composition selon l'une quelconque des revendications 7 à 14, **caractérisée en ce que** la composition contient en outre au moins un polyol.

16. Composition selon la revendication 15, **caractérisée en ce que** le polyol est choisi parmi la glycérine, le sorbitol ou les glycols.

17. Composition selon la revendication 15 ou 16, **caractérisée en ce que** le polyol est présent dans une concentration allant de 5 à 40% et de préférence de 15 à 30% en poids par rapport au poids total de la composition.

18. Composition selon l'une quelconque des revendications 7 à 17, **caractérisée en ce qu'**elle contient en outre au moins une huile siliconée.

19. Composition selon la revendication 18, **caractérisée en ce que** l'huile siliconée est choisie dans le groupe comprenant les silicones volatiles, les polydiméthylsiloxanes, les polyphényltriméthylsiloxanes et les silicones fluorées.

20. Composition selon la revendication 18 ou 19, **caractérisée en ce que** l'huile siliconée est présente dans une concentration allant de 2 à 40%, et de préférence de 10 à 35% en poids par rapport au poids total de la composition.

21. Composition selon l'une quelconque des revendications 7 à 20, **caractérisée en ce que** l'émulsionnant est un diméthicone copolyol ou un alkyldiméthycone copolyol.

22. Composition selon l'une quelconque des revendications 7 à 21, **caractérisée en ce que** l'émulsionnant est présent dans une concentration allant de 0,1 à 10%, et de préférence de 0,5 à 5% en poids par rapport au poids total de la composition.

23. Composition selon l'une quelconque des revendications 7 à 22, **caractérisée en ce qu'**elle contient en outre un ou plusieurs corps gras choisis parmi les cires ou résines de silicone, les huiles fluorées, les huiles d'origine animale, les huiles d'origine végétale, les huiles minérales ou les huiles synthétiques.

24. Composition selon l'une quelconque des revendications 7 à 23, **caractérisée en ce qu'**elle comprend au moins un adjuvant choisi parmi les tensioactifs, les actifs hydrophiles ou lipophiles, les conservateurs, les antioxydants, les solvants, les parfums, les charges, les filtres, les absorbeurs d'odeur, les matières colorantes et les vésicules lipidiques.

25. Composition selon l'une quelconque des revendications 7 à 24, **caractérisée en ce qu'**elle constitue une composition cosmétique et/ou dermatologique.

26. Utilisation d'une composition selon l'une quelconque des revendications 7 à 24 pour un traitement cosmétique de la peau en vue de la tonifier, et la régénérer, de lisser les rides et/ou les ridules de la peau, d'éclaircir le teint, d'éliminer les taches pigmentaires de la peau, et/ou pour lutter contre les méfaits des rayonnement UV, et/ou pour renforcer les tissus cutanés contre les agressions de l'environnement.

27. Utilisation d'une composition selon l'une quelconque des revendications 7 à 25 pour la fabrication d'une crème destinée à un traitement dermatologique.

## Patentansprüche

1. Verwendung eines Silicongummis, der unter den nicht vernetzten, linearen Polydimethylsiloxanen, die gegebenenfalls hydroxyliert oder phenyliert sind, ausgewählt ist, zur Stabilisierung von Ascorbinsäure oder eines Ascorbinsäuresalzes in einer Zusammensetzung zur topischen Anwendung, bei der es sich um eine Wasser-in-Öl-Emulsion oder eine Öl-in-Wasser-Emulsion handelt.

2. Verwendung nach Anspruch 1, **dadurch gekennzeichnet, dass** der Silicongummi unter den Polydiorganosiloxanen mit einer Molmasse im Bereich von 100 000 bis 2 000 000 und insbesondere im Bereich von 100 000 und 1 500 000 ausgewählt ist.

3. Verwendung nach Anspruch 1 oder 2, **dadurch gekennzeichnet, dass** der Silicongummi eine Viskosität von mindestens 0,2 m²/s und vorzugsweise mindestens 0,3 m²/s aufweist.

4. Verwendung nach einem der Ansprüche 1 bis 3, **dadurch gekennzeichnet, dass** der Silicongummi unter den Silicongummis der folgenden Formel (I) ausgewählt ist: 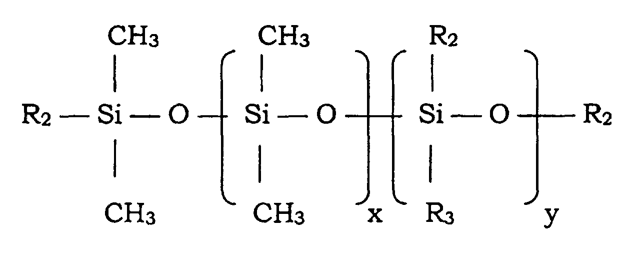 worin bedeuten:
R₂ -CH₃, -OH oder -C₆H₅,
R₃ -CH₃, -OH, -C₆H₅ oder -OSi(CH₃)₃,
x Null oder eine ganze Zahl, und
y eine ganze Zahl, mit der Maßgabe, dass y oder x und y ganze Zahlen sind, die so ausgewählt sind, dass die gewichtsmittlere Molmasse über 100 000 und vorzugsweise im Bereich von 100 000 bis 1 500 000 liegt.

5. Verwendung nach einem der Ansprüche 1 bis 5, **dadurch gekennzeichnet, dass** der Silicongummi unter den Dimethiconen und Dimethiconolen ausgewählt ist.

6. Verwendung nach einem der Ansprüche 1 bis 5, **dadurch gekennzeichnet, dass** der Silicongummi alleine oder im Gemisch mit einem Lösungsmittel verwendet wird, das unter den flüchtigen Siliconen, Polydimethylsiloxanölen, Polyphenylmethylsiloxanölen oder deren Gemischen ausgewählt ist.

7. Zusammensetzung zur topischen Anwendung in Form einer Wasser-in-Öl-Emulsion oder Öl-in-Wasser-Emulsion, **dadurch gekennzeichnet, dass** sie Ascorbinsäure oder eines ihrer Salze, einen Silicongummi, der unter den nicht vernetzten, linearen Polydimethylsiloxanen, die gegebenenfalls hydroxyliert oder phenyliert sind, ausgewählt ist, und ein emulgierendes Silicon enthält und einen pH-Wert im Bereich von 4 bis 7 aufweist.

8. Zusammensetzung nach Anspruch 7, **dadurch gekennzeichnet, dass** der Silicongummi unter den Polydiorganosiloxanen mit einer Molmasse im Bereich von 100 000 bis 2 000 000 und vorzugsweise im Bereich von 100 000 und 1 500 000 ausgewählt ist.

9. Zusammensetzung nach Anspruch 7 oder 8, **dadurch gekennzeichnet, dass** der Silicongummi eine Viskosität von mindestens 0,2 m²/s und vorzugsweise mindestens 0,3 m²/s aufweist.

10. Zusammensetzung nach einem der Ansprüche 7 bis 9, **dadurch gekennzeichnet, dass** der Silicongummi unter den Silicongummis der folgenden Formel (I) ausgewählt ist: 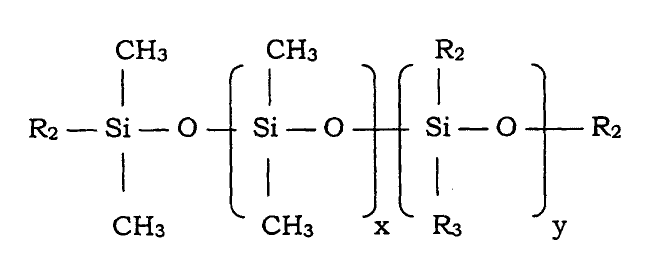 worin bedeuten:
R₂ -CH₃, -OH oder -C₆H₅,
R₃ -CH₃, -OH, -C₆H₅ oder -OSi(CH₃)₃,
x Null oder eine ganze Zahl, und
y eine ganze Zahl, mit der Maßgabe, dass y oder x und y ganze Zahlen sind, die so ausgewählt sind, dass die gewichtsmittlere Molmasse über 100 000 und vorzugsweise im Bereich von 100 000 bis 1 500 000 liegt.

11. Zusammensetzung nach einem der Ansprüche 7 bis 10, **dadurch gekennzeichnet, dass** der Silicongummi unter den Dimethiconen und Dimethiconolen ausgewählt ist.

12. Zusammensetzung nach einem der Ansprüche 7 bis 11, **dadurch gekennzeichnet, dass** der Silicongummi alleine oder im Gemisch mit einem Lösungsmittel verwendet wird, das unter den flüchtigen Siliconen, Polydimethylsiloxanölen, Polyphenylmethylsiloxanölen oder deren Gemischen ausgewählt ist.

13. Zusammensetzung nach einem der Ansprüche 7 bis 12, **dadurch gekennzeichnet, dass** die Ascorbinsäure oder eines ihrer Salze in einer Konzentration von 0,1 bis 20 Gew.-% und vorzugsweise von 2 bis 10 Gew.-%, bezogen auf das Gesamtgewicht der Zusammensetzung, enthalten ist.

14. Zusammensetzung nach einem der Ansprüche 7 bis 13, **dadurch gekennzeichnet, dass** der Silicongummi in einer Wirkstoffkonzentration von 1 bis 20 Gew.-% und vorzugsweise von 2 bis 15 Gew.-%, bezogen auf das Gesamtgewicht der Zusammensetzung, vorliegt.

15. Zusammensetzung nach einem der Ansprüche 7 bis 14, **dadurch gekennzeichnet, dass** die Zusammensetzung ferner mindestens ein Polyol enthält.

16. Zusammensetzung nach Anspruch 15, **dadurch gekennzeichnet, dass** das Polyol unter Glycerin, Sorbit oder Glykolen ausgewählt.

17. Zusammensetzung nach Anspruch 15 oder 16, **dadurch gekennzeichnet, dass** das Polyol in einer Konzentration von 5 bis 40 Gew.-% und vorzugsweise von 15 bis 30 Gew.-%, bezogen auf das Gesamtgewicht der Zusammensetzung, enthalten ist.

18. Zusammensetzung nach einem der Ansprüche 7 bis 17, **dadurch gekennzeichnet, dass** sie ferner mindestens ein Siliconöl enthält.

19. Zusammensetzung nach Anspruch 18, **dadurch gekennzeichnet, dass** das Siliconöl unter den flüchtigen Siliconen, Polydimethylsiloxanen, Polyphenyltrimethylsiloxanen und fluorierten Siliconen ausgewählt ist.

20. Zusammensetzung nach einem der Ansprüche 18 oder 19, **dadurch gekennzeichnet, dass** das Siliconöl in einer Konzentration von 2 bis 40 Gew.-% und vorzugsweise von 10 bis 35 Gew.-%, bezogen auf das Gesamtgewicht der Zusammensetzung, vorliegt.

21. Zusammensetzung nach einem der Ansprüche 7 bis 20, **dadurch gekennzeichnet, dass** der Emulgator ein Dimethiconcopolyol oder ein Alkyldimethiconcopolyol ist.

22. Zusammensetzung nach einem der Ansprüche 7 bis 21, **dadurch gekennzeichnet, dass** der Emulgator in einer Konzentration von 0,1 bis 10 Gew.-% und vorzugsweise von 0,5 bis 5 Gew.-%, bezogen auf das Gesamtgewicht der Zusammensetzung, enthalten ist.

23. Zusammensetzung nach einem der Ansprüche 7 bis 22, **dadurch gekennzeichnet, dass** sie ferner eine oder mehrere Fettsubstanzen enthält, die unter den Siliconwachsen oder Siliconharzen, fluorierten Ölen, Ölen tierischer Herkunft, Ölen pflanzlicher Herkunft, Mineralölen oder synthetischen Ölen ausgewählt sind.

24. Zusammensetzung nach einem der Ansprüche 7 bis 23, **dadurch gekennzeichnet, dass** sie ferner mindestens einen Zusatzstoff enthält, der unter den grenzflächenaktiven Stoffen, hydrophilen oder lipophilen Wirkstoffen, Konservierungsmitteln, Antioxidantien, Lösungsmitteln, Parfums, Füllstoffen, Filtern, Geruchsabsorbern, Farbmitteln und Lipidvesikeln ausgewählt ist.

25. Zusammensetzung nach einem der Ansprüche 7 bis 24, **dadurch gekennzeichnet, dass** es sich um eine kosmetische und/oder dermatologische Zusammensetzung handelt.

26. Verwendung einer Zusammensetzung nach einem der Ansprüche 7 bis 24 zur kosmetischen Behandlung der Haut, insbesondere um die Haut zu stärken, zu regenerieren, die Falten und/ oder Fältchen der Haut zu glätten, den Teint strahlender erscheinen zu lassen, die Pigmentflecken der Haut zu entfernen und/oder die schädlichen Wirkungen der UV-Strahlung zu bekämpfen und/oder das Hautgewebe gegen die Angriffe aus der Umgebung zu stärken.

27. Verwendung einer Zusammensetzung nach einem der Ansprüche 7 bis 25 für die Herstellung einer Creme für die dermatologische Behandlung.

## Claims

1. Use of a silicone gum selected from optionally hydroxyl- or phenyl-containing non-crosslinked linear polydimethylsiloxanes to stabilize ascorbic acid or one of its salts in a topical composition constituting a water-in-oil or oil-in-water emulsion.

2. Use according to Claim 1, **characterized in that** the silicone gum is selected from polydiorganosiloxanes having a molecular mass of between 100,000 and 2,000,000 and, preferably, between 100,000 and 1,500,000.

3. Use according to Claim 1 or 2, **characterized in that** the silicone gum has a viscosity equal to or greater than 0.2 m²/s and, preferably, equal to or greater than 0.3 m²/s.

4. Use according to any one of Claims 1 to 3, **characterized in that** the silicone gum is selected from the silicone gums of formula (I) below: 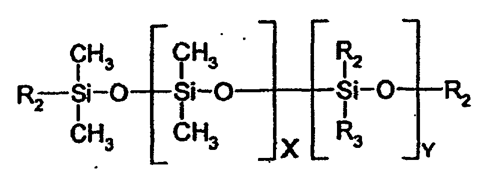 in which:
R₂ represents -CH₃, -OH or -C₆H₅,
R₃ represents -CH₃, -OH, -C₆H₅ or -OSi(CH₃)₃,
x = 0 or an integer, and
y is an integer, with the proviso that y or x and y are integers such that the weight-average molecular mass is greater than 100,000 and preferably between 100,000 and 1,500,000.

5. Use according to any one of Claims 1 to 4, **characterized in that** the silicone gum is selected from dimethicones and dimethiconols.

6. Use according to any one of Claims 1 to 5, **characterized in that** the silicone gum is employed alone or in a mixture with a solvent selected from volatile silicones, polydimethylsiloxane oils, polyphenylmethylsiloxane oils and mixtures thereof.

7. Topical composition in the form of a water-in-oil or oil-in-water emulsion, **characterized in that** it comprises ascorbic acid or one of its salts, a silicone gum selected from optionally hydroxyl- or phenyl-containing non-crosslinked linear polydimethylsiloxanes and a silicon-containing emulsifier and has a pH of between 4 and 7.

8. Composition according to Claim 7, **characterized in that** the silicone gum is selected from polydiorganosiloxanes having a molecular mass of between 100,000 and 2,000,000 and, preferably, between 100,000 and 1,500,000.

9. Composition according to Claim 7 or 8, **characterized in that** the silicone gum has a viscosity equal to or greater than 0.2 m²/s and, preferably, equal to or greater than 0.3 m²/s.

10. Composition according to any one of Claims 7 to 9, **characterized in that** the silicone gum is selected from the silicone gums of formula (I) below: 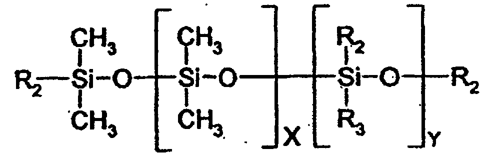 in which:
R₂ represents -CH₃, -OH or -C₆H₅,
R₃ represents -CH₃, -OH, -C₆H₅ or -OSi(CH₃)₃,
x = 0 or an integer, and
y is an integer, with the proviso that y or x and y are integers such that the weight-average molecular mass is greater than 100,000 and preferably between 100,000 and 1,500,000.

11. Composition according to any one of Claims 7 to 10, **characterized in that** the silicone gum is selected from dimethicones and dimethiconols.

12. Composition according to any one of Claims 7 to 11, **characterized in that** the silicone gum is employed alone or in a mixture with a solvent selected from volatile silicones, polydimethylsiloxane oils, polyphenylmethylsiloxane oils and mixtures thereof.

13. Composition according to any one of Claims 7 to 12, **characterized in that** ascorbic acid or one of its salts is present in a concentration ranging from 0.1 to 20% and, preferably, from 2 to 10% by weight relative to the total weight of the composition.

14. Composition according to any one of Claims 7 to 13, **characterized in that** the silicone gum is present in an active-substance concentration ranging from 1 to 20% and, preferably, from 2 to 15% by weight relative to the total weight of the composition.

15. Composition according to any one of Claims 7 to 14, **characterized in that** the composition additionally comprises at least one polyol.

16. Composition according to Claim 15, **characterized in that** the polyol is selected from glycerol, sorbitol and glycols.

17. Composition according to Claim 15 or 16, **characterized in that** the polyol is present in a concentration ranging from 5 to 40% and, preferably, from 15 to 30% by weight relative to the total weight of the composition.

18. Composition according to any one of Claims 7 to 17, **characterized in that** the composition additionally comprises at least one silicone oil.

19. Composition according to Claim 18, **characterized in that** the silicone oil is selected from the group consisting of volatile silicones, polydimethylsiloxanes, polyphenyltrimethylsiloxanes and fluorinated silicones.

20. Composition according to Claim 18 or 19, **characterized in that** the silicone oil is present in a concentration ranging from 2 to 40% and, preferably, from 10 to 35% by weight relative to the total weight of the composition.

21. Composition according to any one of Claims 7 to 20, **characterized in that** the emulsifier is a dimethicone copolyol or an alkyldimethicone copolyol.

22. Composition according to any one of Claims 7 to 21, **characterized in that** the emulsifier is present in a concentration ranging from 0.1 to 10% and, preferably, from 0.5 to 5% by weight relative to the total weight of the composition.

23. Composition according to any one of Claims 7 to 22, **characterized in that** it additionally comprises one or more fats selected from silicone resins or waxes, fluorinated oils, oils of animal origin, oils of plant origin, mineral oils and synthetic oils.

24. Composition according to any one of Claims 7 to 23, **characterized in that** it comprises at least one adjuvant selected from surfactants, hydrophilic or lipophilic active agents, preservatives, antioxidants, solvents, perfumes, fillers, screening agents, odour absorbers, colorants and lipid vesicles.

25. Composition according to any one of Claims 7 to 24, **characterized in that** it constitutes a cosmetic and/or dermatological composition.

26. Use of a composition according to any one of Claims 7 to 24 for a cosmetic treatment of the skin with a view to toning it and regenerating it, smoothing out the lines and/or wrinkles of the skin, lightening the complexion, removing pigmentary blemishes from the skin, and/or combating the damage done by UV radiation, and/or strengthening the cutaneous tissues against environmental attack.

27. Use of a composition according to any one of Claims 7 to 25 for the preparation of a cream intended for dermatological treatment.
